# EUROPEAN PATENT APPLICATION

(11) **EP 4 091 593 A1**
(43) Date of publication of application: **23.11.2022**
(21) Application number: 21175188.8
(22) Date of filing: 21.05.2021
(51) Int. Cl.: A61F 13/551

(54) **A PACKAGE OF FEMININE HYGIENE PRODUCTS**

(71) Applicant: Fameccanica.Data S.p.A., 66020 San Giovanni Teatino (CH) (IT)
(72) Inventor: OLIVIERI, Paola, I-66020 San Giovanni Teatino (Chieti) (IT)
(74) Representative: Marchitelli, Mauro

(57) **Abstract**

A package of feminine hygiene products (10) comprising a casing (12) containing a plurality of feminine hygiene products (14, 16, 18, 20) arranged in a plurality of groups (22, 24, 26, 28), wherein the feminine hygiene products (14, 16, 18, 20) of each group (22, 24, 26, 28) have an absorption capacity different from the absorption capacity of the feminine hygiene products (14, 16, 18, 20) of the other groups (22, 24, 26, 28).

## Description

### Field of the invention

The present invention relates in general to packaging of absorbent sanitary articles and specifically to packaging of feminine hygiene products, such as feminine sanitary pads, pantyliner, etc.

### Description of the prior art

A feminine hygiene product is an absorbent item worn in the underwear when menstruating, bleeding after giving birth, recovering from gynecologic surgery, experiencing a miscarriage or abortion, or in any other situation where it is necessary to absorb a flow of blood. A menstrual pad is a type of feminine hygiene product that is worn externally, unlike tampons which are worn internally. Pads are generally changed by being stripped off the pants and panties, taking out the old pad, sticking the new one on the inside of the panties and pulling them back on. Pads are recommended to be changed after a certain number of hours to avoid certain bacteria that can fester in blood.

Feminine hygiene products are made from a range of materials, differing depending on style, country of origin, and brand. The feminine hygiene pads are not the same as incontinence pads, which generally have higher absorbency and are worn by people who have urinary incontinence problems or experience stress incontinence.

There are several different types of disposable feminine hygiene pads:
- Panty liner: designed to absorb daily vaginal discharge, light menstrual flow, "spotting", slight urinary incontinence, or as a backup for tampon or menstrual cup use;
- Ultra-thin: a very compact and thin pad, which may be as absorbent as a Regular or Maxi/Super pad but with less bulk;
- Regular: a middle range absorbency pad;
- Maxi/Super: a larger absorbency pad, useful for the start of the menstrual cycle when menstruation is often heaviest;
- Overnight: a longer pad to allow for more protection while the wearer is lying down, with an absorbency suitable for overnight use.

The shape, absorbency and lengths may vary depending on manufacturers, but usually range from the short slender panty liner to the larger and longer overnight. Other options are often offered in a manufacturer's line of pads, such as wings or tabs that wrap around the sides of underwear to add additional leak protection and help secure the pad in place.

Different types of feminine hygiene pads have different absorption capacity. Various methods are in use to measure the absorption capacity of absorbent sanitary products. For instance, the standard ISO 11948-1:1996 gives a method for determining the absorption capacity of the absorbent core of body-worn urine-absorbing aids.

Feminine hygiene pads are packaged in casings which may be made by a flexible sheet of packaging material (e.g. of paper or plastic) or by a cardboard box. Each packaging contains a predetermined number of feminine hygiene pads (e.g. 16 pads). In eack package the individual feminine hygiene pads may be packaged in primary packages (pouches) made of a flexible sheet of paper or plastic.

At present, each package contains feminine hygiene pads of the same type, having all the same size and consequently the same absorption capacity.

During the period, the intensity of the menstrual flow varies considerably from a more intense flow during the initial days to very light bleeding in the final days. Accordingly, the user should have available a number of different packages of hygiene pads with different sizes and absorption capacity. Normally a user should have two or three packages for a single cycle, which may remain unfinished or finish too early and may create space problems or unavailability of certain types of hygiene pads. This problem is more acute for frequent travellers.

It is quite common that users may use the same type of hygiene pads for the whole duration of the cycle, which inevitably leads to using in many days hygiene pads having an absorption capacity which is not proportioned to the intensity of the menstrual flow, i.e. which is either too high or too low with respect to the intensity of the menstrual flow.

### Object and summary of the invention

The object of the present invention is to provide a package of feminine hygiene products which overcomes the problems of the prior art.

According to the present invention, this object is achieved by a package of feminine hygiene products having the features of claim 1.

Preferred embodiments of the invention form the subject of the dependent claims.

The claims form an integral part of the technical teaching provided herein in relation to the invention.

### Brief description of the drawings

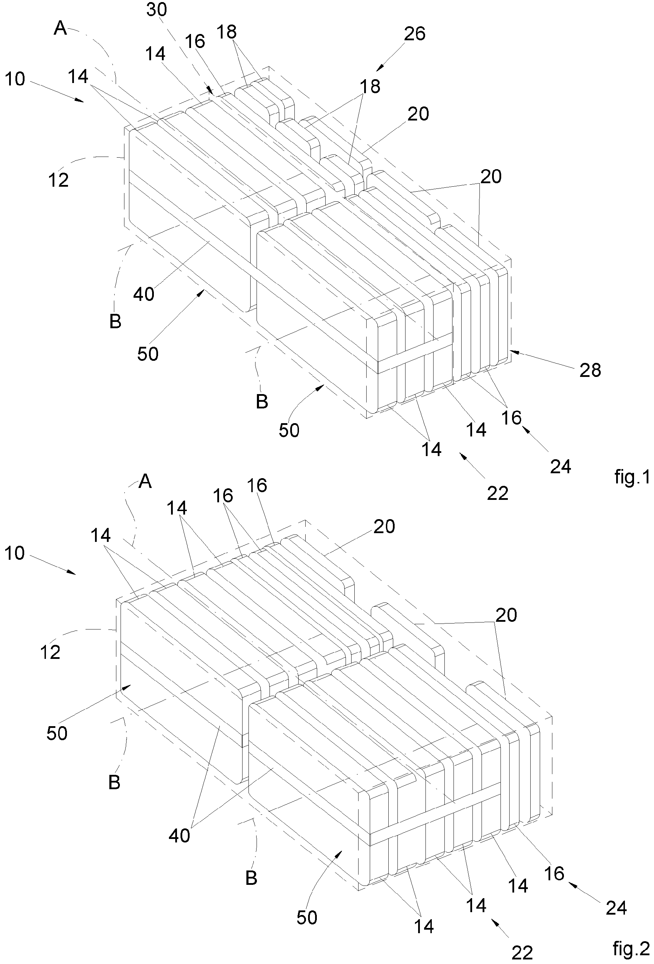

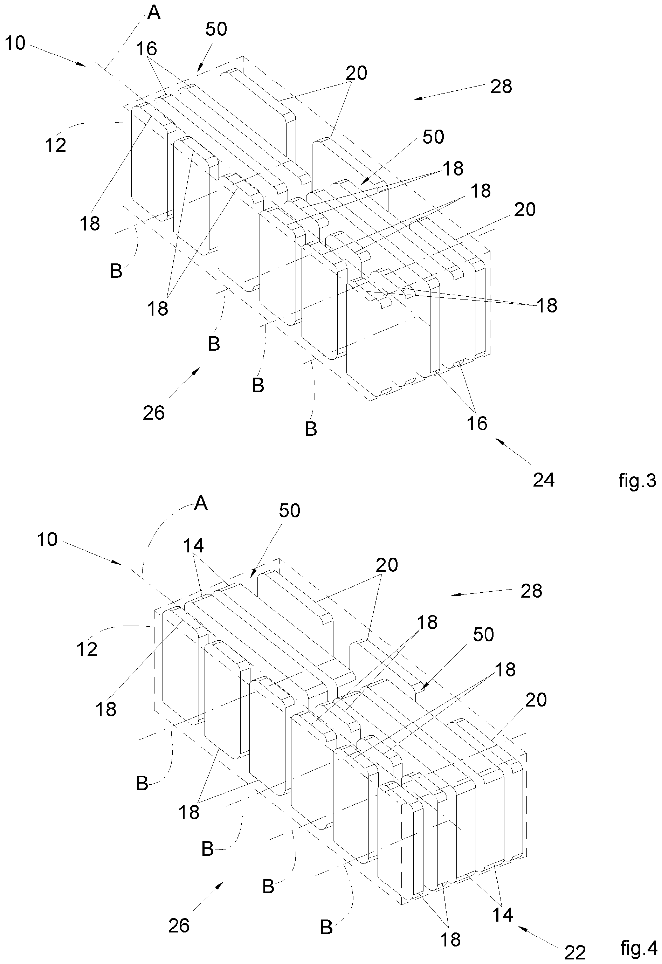

The invention will now be described, purely by way of non-limiting example, with reference to the annexed drawings, wherein figures 1-6 are schematic perspective view of possible embodiments of packages according to the present invention.

### Detailed description

With reference to figures 1-4 a package of feminine hygiene products is indicated by the reference number 10.

The package 10 comprises a casing 12, which may be formed by a flexible sheet of packaging material, such as paper or plastic material or by a cardboard box.

The casing 12 contains a plurality of feminine hygiene products 14, 16, 18, 20 having different absorption capacity.

The feminine hygiene products 14, 16, 18, form a plurality of groups 22, 24, 26, 28. Each group 22, 24, 26, 28 comprises a plurality of feminine hygiene products 14, 16, 18, 20 having the same absorption capacity. The feminine hygiene products 14, 16, 18, 20 of each group 22, 24, 26, 28 have an absorption capacity different from the absorption capacity of the feminine hygiene products 14, 16, 18, 20 of the other groups 22, 24, 26, 28.

In a possible embodiment, the feminine hygiene products 14 of the first group 22 are hygiene pads with a high absorption capacity, having an absorption capacity according to the standard ISO 11948-1 comprised between 620 g and 1000 g.

In a possible embodiment, the feminine hygiene products 16 of the second group 24 are hygiene pads with a medium-high absorption capacity, having an absorption capacity according to the standard ISO 11948-1 comprised between 360 g and 619 g.

In a possible embodiment, the feminine hygiene products 18 of the third group 26 are hygiene pads with a medium absorption capacity, having an absorption capacity according to the standard ISO 11948-1 comprised between 151 g and 359 g.

In a possible embodiment, the feminine hygiene products 20 of the fourth group 28 are hygiene pads with a low absorption capacity (pantyliners), having an absorption capacity according to the standard ISO 11948-1 comprised between 90 g and 150 g.

Each group 22, 24, 26, 28 comprises a number of feminine hygiene products 14, 16, 18, 20 comprised between 3 and 9.

The total number of feminine hygiene products 14, 16, 18, 20 in each package 10 may be 16, which is the standard number of items in the conventional packages of feminine hygiene products.

The configuration of the packages 10 may vary so as to provide packages adapted to the needs of different users. In the following some possible package configurations are disclosed. Some of the package configurations may contain 16 sanitary pads per package, which is the number of hygiene pads contained in the current standard packages.

Figure 1 shows a package configuration for users having an irregular flow, which consists of six pads 14 with high absorption capacity, three pads 16 with medium-high absorption capacity, four pads 18 with medium absorption capacity, and three pads 20 with low absorption capacity.

Figure 2 shows a package configuration for users having an abundant constant flow, which consists of nine pads 14 with high absorption capacity, four pads 16 with medium-high absorption capacity, and three pads 20 with low absorption capacity.

Figure 3 shows a package configuration for users having an average constant flow, which consists of four pads 16 with medium-high absorption capacity, nine pads 18 with medium absorption capacity, and three pads 20 with low absorption capacity.

Figure 4 shows a package configuration for users having a constant flow not very abundant, which consists of four pads 14 with high absorption capacity, nine pads 18 with medium absorption capacity, and three pads 20 with low absorption capacity.

The pads 14, 16, 18, 20 may be arranged in various way in the package 10. Each pad 14, 16, 18, 20 has a flat rectangular shape with two parallel main faces and a thickness which is small as compared to the dimensions of the main faces. The package 10 has a parallelepided shape elongated along a main direction A. In a possible embodiment, the pads 14, 16, 18, 20 may be arranged in the package 10 with the respective main faces parallel to the main direction A as shown in figures 1 to 4. In a possible embodiment, the pads 14, 16, 18, 20 may be arranged in the package 10 with the respective main faces orthogonal to the main direction A.

A plurality of pads 14, 16, 18, 20 of the same type may be stacked to form one or more packs 50, each containing two or more pads 14, 16, 18, 20 of the same type overlapped to each other along a stacking direction B orthogonal to the main faces of the pads 14, 16, 18, 20. The pads 14, 16, 18, 20 of each group 22, 24, 26, 28 may be arranged in one or more packs 50.

In a possible embodiment, the package 10 may contain two or more packs 50 arranged with the respective stacking directions B parallel to each other and orthogonal to the main direction A, as shown in figures 1-4.

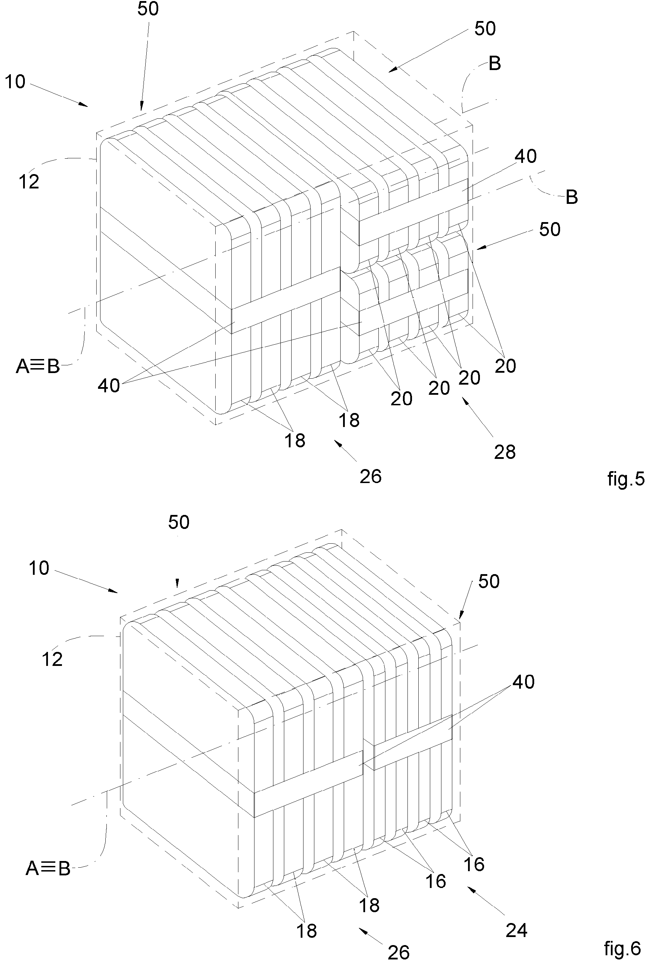

In a possible embodiment, the package 10 may contain two or more packs 50 arranged with the respective stacking directions B parallel to each other and parallel to the main direction A, as shown in figures 5 and 6.

In a possible embodiment, a plurality of pads 14, 16, 18, 20 of the same type may be bound together by a band 40 of paper or plastic material. In a possible embodiment, the band 40 may wrap the pads 14, 16, 18, 20 of two packs 50 set side by side, as shown in figure 1. In a possible embodiment, the band 40 may wrap all the pads 14, 16, 18, 20 of the same pack 50 as shown in figures 2, 5 and 6.

A separator 30 (figure 1), formed for instance by a cardboard sheet, may be placed between two adjecent groups 22, 24, 26, 28. The separator may contain a visual indication of the type of pads of each group, to make it easier for the user to identify the different pads.

Alternatively, the package 10 may comprise a separator every 3 or 4 hygiene pads 14, 16, 18, 20, indicating the day of use (Day 1, Day 2, ... Day 5).

Of course, without prejudice to the principle of the invention, the details of construction and the embodiments may vary widely with respect to what has been described and illustrated herein, without thereby departing from the scope of the invention as defined by the claims that follow.

## Claims

1. A package of feminine hygiene products (10) comprising:
- a casing (12), and
- a plurality of feminine hygiene products (14, 16, 18, 20) contained in said casing (12),
wherein said feminine hygiene products (14, 16, 18, 20) are arranged in a plurality of groups (22, 24, 26, 28), wherein each group (22, 24, 26, 28) comprises a plurality of feminine hygiene products (14, 16, 18, 20) having the same absorption capacity, and wherein the feminine hygiene products (14, 16, 18, 20) of each group (22, 24, 26, 28) have an absorption capacity different from the absorption capacity of the feminine hygiene products (14, 16, 18, 20) of the other group(s) (22, 24, 26, 28).

2. The package of claim 1, comprising at least three groups (22, 24, 26, 28) of feminine hygiene products (14, 16, 18, 20) having different absorption capacity.

3. The package of claim 1 or claim 2, comprising a first group (22) of feminine hygiene products (14) having an absorption capacity according to the standard ISO 11948-1 comprised between 620 g and 1000 g.

4. The package of any of the preceding claims, comprising a second group (24) of feminine hygiene products (16) having an absorption capacity according to the standard ISO 11948-1 comprised between 360 g and 619 g.

5. The package of any of the preceding claims, comprising a third group (26) of feminine hygiene products (18) having an absorption capacity according to the standard ISO 11948-1 comprised between 359 g and 151 g.

6. The package of any of the preceding claims, coprising a fourth group (28) of feminine hygiene products (20) having an absorption capacity according to the standard ISO 11948-1 comprised between 90 g and 150 g.

7. The package of any of the preceding claims, wherein each of said groups (22, 24, 26, 28) comprises a number of feminine hygiene products (14, 16, 18, 20) comprised between 3 and 9.

8. The package of any of the preceding claims, comprising at least one separator (30) placed between two different groups (22, 24, 26, 28)

9. The package of any of the preceding claims, wherein the package (10) has a parallelepided shape elongated along a main direction (A), wherein said pads (14, 16, 18, 20) are arranged in the package (10) with respective main faces parallel or orthogonal to said main direction (A).

10. The package of claim 9, wherein a plurality of pads (14, 16, 18, 20) of the same type are stacked to form one or more packs (50), each pack (50) containing two or more pads (14, 16, 18, 20) of the same type overlapped to each other along a stacking direction (B) orthogonal to the main faces of the pads (14, 16, 18, 20).

11. The package of claim 10, comprising two or more packs (50) arranged with the respective stacking directions (B) parallel to each other and orthogonal to said main direction A.

12. The package of claim 10, comprising two or more packs (50) arranged with the respective stacking directions (B) parallel to each other and parallel to said main direction (A).

13. The package of any of claims 9-12, wherein a plurality of pads (14, 16, 18, 20) of the same type are bound together by a band (40).

14. The package of claim 13, wherein said band (40) wraps the pads (14, 16, 18, 20) of two packs (50) set side by side.

15. The package of claim 13, wherein said band (40) wraps all the pads (14, 16, 18, 20) of the same pack (50).
